# EUROPEAN PATENT SPECIFICATION

(11) **EP 2 696 817 B1**
(45) Date of publication and mention of the grant of the patent: **03.06.2015**
(21) Application number: 12714307.1
(22) Date of filing: 11.04.2012
(51) Int. Cl.: A61F 5/445, A61F 5/443

(54) **AN OSTOMY POUCH**
OSTOMIEBEUTEL
POCHE D'OSTOMIE

(30) Priority: 12.04.2011 EP 11162035
(43) Date of publication of application: 19.02.2014
(73) Proprietor: Hollister Incorporated, Libertyville, IL 60048-3781 (US)
(72) Inventor: RICHMANN, Sussie, 3150 Hellebæk (DK); GRUM-SCHWENSEN, Christen, 3400 Hillerød (DK)
(74) Representative: Høiberg A/S
(86) International application number: PCT/EP2012/056520
(87) International publication number: WO 2012/140040

(56) References cited:
- EP-A1- 2 229 924
- WO-A2-2007/134608
- WO-A2-2008/150991

## Description

The present disclosure relates to an ostomy pouch comprising a first proximal pouch wall and a second distal pouch wall joined together along the outer periphery to form a cavity for accommodating waste material, an inlet provided in the first pouch wall for receiving waste into the pouch, and a comfort layer provided at least on the distal side wall.

When applying an ostomy pouch, it is important that the pouch is correctly positioned around the stoma. In order to ensure proper positioning it is desirable for the user to inspect the stoma. As the stoma will be covered by the pouch, this inspection can take place by looking through an ostomy pouch with transparent side walls or at least a transparent portion of the side walls. Examples of such pouches are known from e.g. US 3,570,490 and WO 2008/150991.

In the pouch disclosed in WO 2008/150991, which is regarded as the closest prior art, two panels are provided, one panel covering on the top of the pouch and a second panel covering the lower part of the pouch. These two panels are slightly overlapping without being sealed to each other across the pouch. This arrangement allows the user to manually separate the overlap to inspect the condition of the stoma.

Although this allows for visual inspection of the stoma, there are some drawbacks associated with this. During the application of the pouch onto the user's skin it can be difficult for the user to see what he or she is doing since the user will be looking at the stoma area from the top. It is most likely that the user will position the pouch and inspect the stoma condition via a mirror. This however may be somewhat inconvenient and difficult as this only gives the user an indirect view of the stoma and the pouch. In some situations the user may be assisted where it may be a nurse or other healthcare staff who will inspect and change the ostomy pouch. In this situation the assistant will have a better direct view, but he or she is not always available for the ostomy pouch user. So although visible inspection is possible it is inconvenient for the user to do.

In view of these drawbacks it is an object of the present disclosure to provide an ostomy pouch which is easier to apply and allows for easier inspection of the stoma. This object is achieved by an ostomy pouch of the initially mentioned kind wherein the ostomy pouch comprises a first proximal pouch wall and a second distal pouch wall joined together along their outer periphery to form a cavity for accommodating waste material, an inlet provided in the first pouch wall for receiving waste into the pouch, a comfort layer provided at least on the distal side wall and wherein the comfort layer comprises two cover panels covering each side of the pouch and sealed along the periphery wherein said two cover panels abut or overlap each other without being attached to one another along a longitudinal centre line of the pouch.

By providing this vertical split in the comfort layer by providing the two cover panels beside each other preferably with a slight overlap, the user can actually inspect the stoma by separating the two panels from each other since the vertical split makes the stoma viewable from above. By providing a vertical split rather than a horizontal split, it is found advantageous since the comfort layer backing does not tend to "open" when the pouch is filled and bulges out.

This is not only advantageous during application of the ostomy pouch and when inspecting the condition of the stoma. Besides inspecting the condition of the stoma, the present ostomy pouch is also advantageous by making it possible to inspect if the adhesive is being dissolved. The pouch must be changed after a while and before the adhesive attaching the pouch directly or indirectly to the skin starts to wear off as stoma effluents dissolves or penetrate into the adhesive and thereby reduces the adhesive effect. This adhesion wear out starts from the inside of the pouch and it is realised that that by an ostomy pouch according to the present disclosure it is easy also to inspect the condition of the inner edge of the adhesive wafer.

It is preferred that the distal pouch wall is transparent or translucent so that there is an unobstructed view through the distal wall to inspect the inside of the pouch and the area around the opening on proximal side wall.

Preferably, both of the comfort layer panels are joined to the distal pouch wall along the periphery of the pouch. Hereby, the comfort layer is presented as an integrated structure of the pouch leaving the vertical split along the pouch to inspect the stoma. The cover panels of the comfort layer may advantageously comprise a non-woven film. Moreover, the cover panels further may comprise an opaque film as an inner film and the non-woven film as the outer film. Hereby, the cover is provided with a comfortable outer appearance and the cover is less transparent so that the content of the pouch is kept concealed. To enhance this effect, the proximal side wall may furthermore be made of an opaque film, and preferably covered with a comfort layer of a non-woven panel.

In an embodiment of the disclosure the pouch comprises an adhesive wafer on the proximal side wall for adhesively securing the pouch to the user's skin. Besides such one-piece ostomy appliance, it is realised that the present disclosure may also be used in relation to a two-piece ostomy appliance.

In a further embodiment, the wafer comprises a starter hole surrounded by a plurality of cutting guidelines for enabling the person to cut the hole into a desired aperture size, where the cutting guidelines substantially converge with the starter hole at an upper region of the hole, i.e. at a radial corresponding to the longitudinal centre line of the pouch. Depending on the size of the stoma hole the position of the hole may vary. By a vertical split in the backing cover, the possibility of inspection of the stoma remains the same irrespective of the size and thereby the position of the stoma opening.

In one embodiment, the pouch is provided with an outlet portion with an outlet for draining the content of the pouch. As an alternative to a drainable pouch, the pouch may be a disposable (closed) pouch.

In the following the disclosure is described in more detail with reference to the accompanying drawings, in which:
Fig. 1 is a distal side view of a pouch according to a first embodiment;
fig. 2 is a schematic cross-section view through the line A-A in fig. 1;
fig. 3 is a proximal side view of a pouch according to a first embodiment;
fig. 4 is a distal side view of a pouch according to a second embodiment; and
fig. 5 is a proximal side view of same.

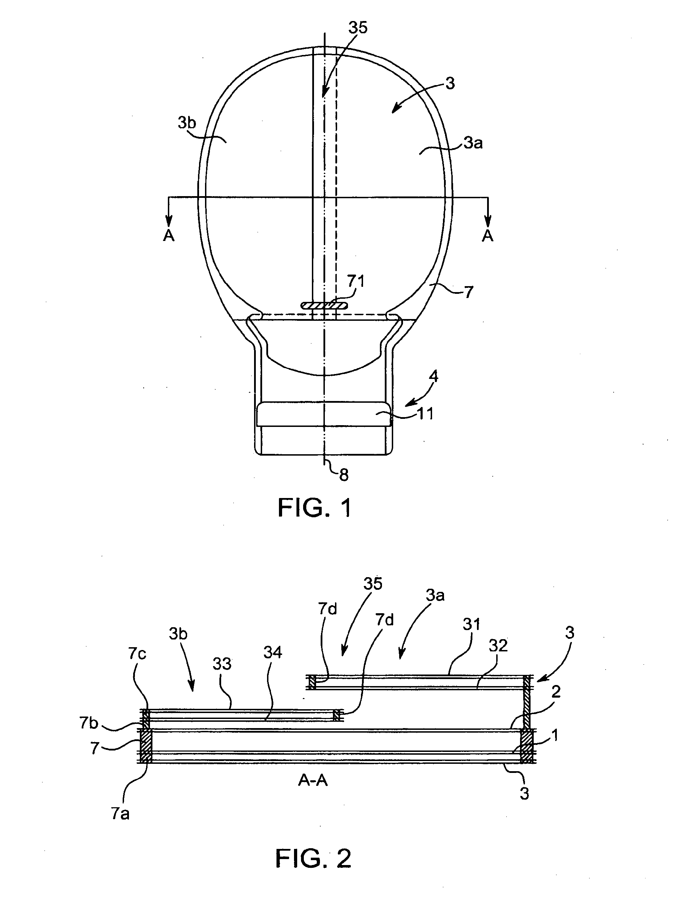

With reference to figures 1 and 2, the drainable ostomy pouch is an assembly of two sidewalls 1 and 2 where the proximal sidewall 1 is provided with an inlet 14 and the proximal sidewall 1 and the distal sidewall 2 which according to the embodiment shown are provided with a dressing panel or comfort layer 3. The side walls 1,2 may be made of any suitable material, such as a flexible plastic material.

The sidewalls 1, 2 as well as the comfort layers 3 on each side are joined along their edges by a peripheral welding 7 to form a cavity between the two sidewalls 1, 2. The sidewalls 1, 2 are formed with a main portion for providing the cavity for accommodating waste material entering the pouch and with a downwardly extending outlet portion 4 which terminate in a discharge opening 13. The peripheral welding 7 accordingly leaves the discharge opening without any welding. The peripheral welding 7 is thus formed with a horseshoe-like form defining and following the contour of the pouch.

As shown in figures 1 and 2, the outlet portion 4 is provided with a transversely extending bias member 10 and 11 on each of the two sidewalls 1, 2. The bias members 10, 11 are preferably in the form of relative stiff but flexible, spring-like strips. The bias members 10, 11 may be flat or slightly curved when they are in their resting position abutting each other. In order to facilitate the opening, the flat or slightly curved bias members 10,11 may be provided with score lines (not shown).

The distal side wall 2 is made of a transparent film, such as a clear film, and the proximal sidewall 1 is made of an opaque film, such as a beige film. The comfort layer 3 on the proximal side is a non-woven film layer. On the distal side, the comfort layer 3 is provided as two side panels 3a, 3b which are secured to the pouch side wall 2 along the peripheral edge of the pouch at the pouch seal 7.

The two side panels 3a and 3b abut or form an overlap 35 along a longitudinally oriented line in the pouch. In one embodiment, the overlap 35 is along a longitudinal centre line 8 of the pouch. As shown in fig. 1, the two side panels 3a, 3b may be provided with a cross sealing 71 sealing only the two side panels 3a, 3b together in the overlap region. Hereby, the lower edges of the two side panels 3a, 3b are held together.

With reference to figure 2 which is a schematic cross-sectional view along the line A-A in fig. 1, the pouch is made up by an opaque, such as beige, proximal side wall 1 which is sealed along the pouch periphery to the clear distal side wall 2 by a peripheral pouch seal 7. On the proximal side, a non-woven comfort layer 3 is provided and peripherally sealed to the pouch at the seal 7a. On the distal side, the comfort layer 3 is provided as a first side panel 3a and a second side panel 3b, which are provided with an overlap 35 at the centre of the pouch. The side panels 3a, 3b are overlapping but not sealed to each other at the overlap 35 but are sealed along the periphery at the seal 7b. The side panels 3a, 3b are made of an outer, non-woven layer 31, 33 and an inner film 32, 34 which is opaque, preferably beige. The comfort layer panels 3a, 3b are heat sealed together at the peripheral sealing 7c and the centre sealing 7d.

From fig. 2 it is apparent that the peripheral sealings, i.e. the pouch sealing 7 and the sealings 7a, 7b and 7c are simultaneously positioned.

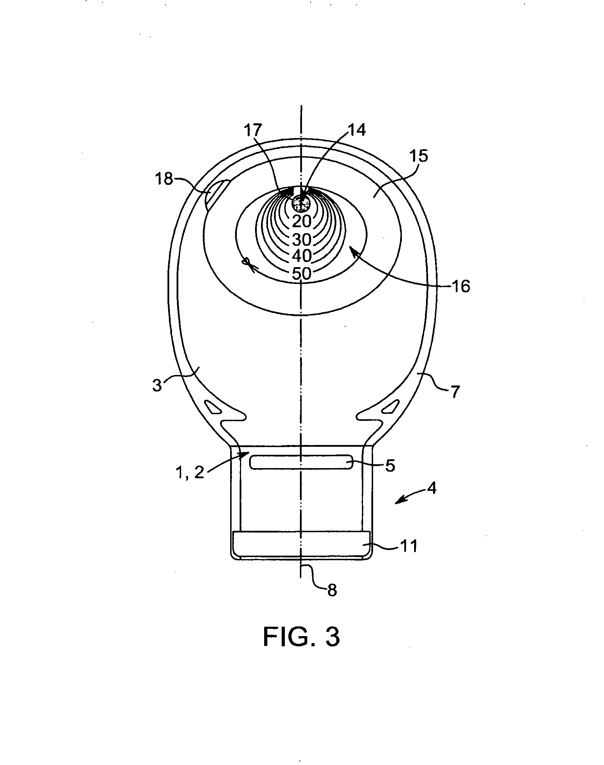

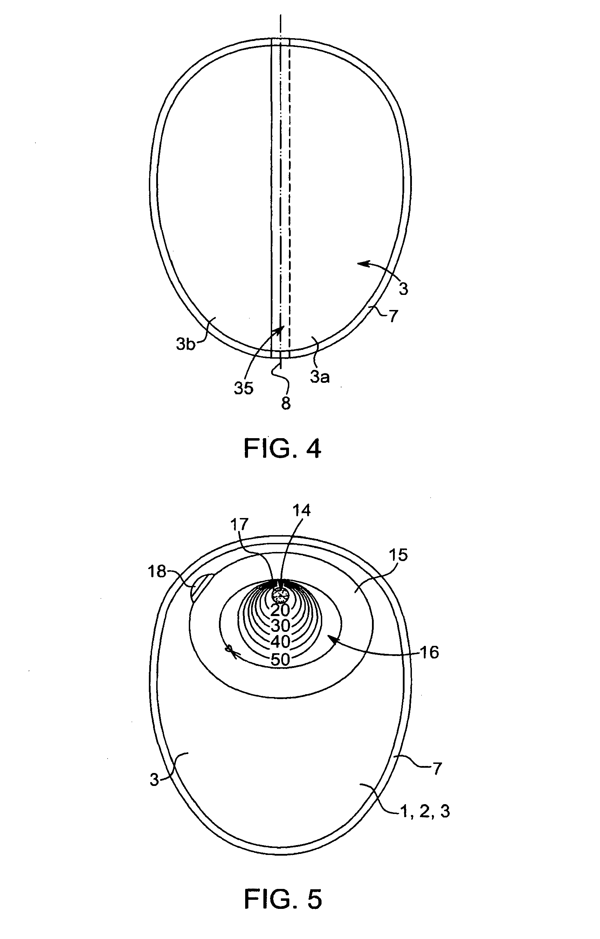

In fig. 3 and 5 examples of a pouch according to a first embodiment of the disclosure is shown. In these figures the pouches are shown from the body side. The product is provided with an inlet 14. Around the inlet there is provided an adhesive wafer or barrier material 15 for adhesively attaching the pouch to the skin of a user or to a face plate pre-mounted around the stoma of the user, when a release liner 18 is removed. The adhesive wafer 15 is provided with a small starter hole 17 and annular cutting guidelines 16 around the starter hole 17 printed on the wafer or printed on a release liner provided on the body side of the wafer, so that the user can prepare the adhesive wafer to fit the actual size of the stoma by cutting the inlet opening 14 into the right size.

The pouch according to the embodiment of figures 3 and 5 is provided with an outlet portion 4, which is provided with transversely extending bias members 11 on each of the two sidewalls 1, 2. The bias members 11 are preferably in the form of relative stiff but flexible, spring-like strips. The drainage portion 4 may be rolled around these bias member strips 11 and secured to a fastening strip 5 for closure of the pouch.

By the embodiments according to the disclosure shown in figures 3 and 5, there is provided an off-centre starter hole 17 as it is realized that when the starter hole 17 is located off-centre rather than in the middle of the barrier 15 it is possible to locate the pouch lower on the body of the user and at the same time achieve a larger "active" volume as the head space in the pouch is decreased.

This is advantageous from a user's perspective as the user wants discrete pouches which can be hidden under the clothing. A low head space and a relatively smaller pouch which can be located lower on the user's body are achievable by the disclosure. Furthermore, a low fit also means that opening the pouch according to the first embodiment of fig. 1 will be easier and consequently the pouch will be easier to empty since the outlet opening 4 will be closer to the toilet.

Another advantage of achieving this lower head space is that this also results in a less prolapse of the pouch, i.e. the pouch will not hang as much outwards from the body when weight is applied.

Figures 4 and 5 show a second embodiment of a pouch with an adhesive wafer and a vertically split comfort layer having two side panels 3a, 3b on the distal side as described above. In this embodiment, however, the pouch is a disposable (closed) pouch. Therefore, the peripheral sealing 7 is uninterrupted along the entire periphery of the pouch. The two side panels 3a, 3b are arranged with a slight overlap 35 where the two panels 3a, 3b are not joined to each other.

Although the figures show pouches with an adhesive wafer for attachment directly onto the skin, i.e. a so-called one piece product, it is realized that the above described off-center starter hole solution may be used in relation to both one piece as well as two-piece ostomy appliances.

## Claims

1. An ostomy pouch comprising
a first proximal pouch wall (1) and a second distal pouch wall (2) joined together along their outer periphery to form a cavity for accommodating waste material;
an inlet (14) provided in the first pouch wall (1) for receiving waste into the pouch;
a comfort layer (3) provided at least on the distal pouch wall (2);
the comfort layer (3) comprises two cover panels (3a, 3b) covering each side of the pouch and sealed along the periphery wherein said two cover panels (3a, 3b) abut or overlap each other without being permanently attached to one another;
**characterized in that**
said two cover panels (3a, 3b) abut or overlap each other without being permanently attached to one another along a longitudinally oriented line (8) on the pouch.

2. A pouch according to claim 1, wherein the distal pouch wall (2) is transparent or translucent.

3. A pouch according to claim 1 or 2, wherein both of the cover panels (3a, 3b) are joined to the distal pouch wall (2) along the periphery of the pouch.

4. A pouch according to any of the preceding claims, wherein the cover panels (3a, 3b) of the comfort layer comprise a non-woven film.

5. A pouch according to claim 4, wherein the cover panels (3a, 3b) further comprises an opaque film as an inner film and the non-woven film as the outer film.

6. A pouch according to any of the preceding claims, wherein the proximal side wall (1) is made of an opaque film, and preferably covered with a comfort layer of a non-woven panel.

7. A pouch according to any of the preceding claims, wherein the pouch comprises an adhesive wafer (15) on the proximal side wall (1) for adhesively securing the pouch to the user's skin.

8. A pouch according to claim 7, wherein said wafer (15) comprises a starter hole (17) surrounded by a plurality of cutting guidelines (16) for enabling the person to cut the hole into a desired aperture size, where the cutting guidelines (16) substantially converge with the starter hole (17) at an upper region of the hole (17), i.e. at a radial corresponding to the longitudinal centre line of the pouch.

9. A pouch according to any of the preceding claims, wherein the pouch is provided with an outlet portion (4) with an outlet for draining the content of the pouch.

## Patentansprüche

1. Stomabeutel, der umfasst:
eine erste nahe Beutelwand (1) und eine zweite ferne Beutelwand (2), die miteinander entlang ihres Außenumfang verbunden sind, um einen Hohlraum zum Aufnehmen von Ausscheidungsmaterialien zu bilden,
einen Einlass (14), der in der ersten Beutelwand (1) vorgesehen ist, um Ausscheidungen in den Beutel aufzunehmen,
eine Komfortschicht (3), die wenigstens an der fernen Beutelwand (2) vorgesehen ist,
wobei die Komfortschicht (3) zwei Deckpaneele (3a, 3b) aufweist, die beide Seiten des Beutels bedecken und entlang des Umfangs abgedichtet sind, wobei die zwei Deckpaneele (3a, 3b) aneinander anstoßen oder einander überlappen, ohne permanent aneinander befestigt zu sein,
**dadurch gekennzeichnet, dass:**
die zwei Deckpaneele (3a, 3b) entlang einer in der Längsrichtung ausgerichteten Linie (8) an dem Beutel aneinander anstoßen oder einander überlappen, ohne permanent aneinander befestigt zu sein.

2. Beutel nach Anspruch 1, wobei die ferne Beutelwand (2) transparent oder durchscheinend ist.

3. Beutel nach Anspruch 1 oder 2, wobei beide Deckpaneele (3a, 3b) mit der fernen Beutelwand (2) entlang des Umfangs des Beutels verbunden sind.

4. Beutel nach einem der vorstehenden Ansprüche, wobei die Deckpaneele (3a, 3b) der Komfortschicht eine nicht-gewebte Folie umfassen.

5. Beutel nach Anspruch 4, wobei die Deckpaneele (3a, 3b) weiterhin eine opake Folie als eine innere Folie und die nicht-gewebte Folie als die äußere Folie umfassen.

6. Beutel nach einem der vorstehenden Ansprüche, wobei die nahe Seitenwand (1) aus einer opaken Folie ausgebildet ist und vorzugsweise durch eine Komfortschicht aus einem nichtgewebten Paneel bedeckt ist.

7. Beutel nach einem der vorstehenden Ansprüche, wobei der Beutel eine Klebefläche (15) an der nahen Seitenwand (1) für das klebende Befestigen des Beutels an der Haut eines Benutzers umfasst.

8. Beutel nach Anspruch 7, wobei die Fläche (15) ein Starterloch (17) umfasst, das durch eine Vielzahl von Schneideführungslinien (16) umgeben ist, damit eine Person das Loch zu einer gewünschten Öffnungsgröße schneiden kann, wobei die Schneideführungslinien (16) im Wesentlichen mit dem Starterloch (17) an einem oberen Bereich des Lochs (17) zusammenlaufen, d.h. an einer Radiallinie, die der Längsmittenlinie des Beutels entspricht.

9. Beutel nach einem der vorstehenden Ansprüche, wobei der Beutel mit einem Auslassteil (4) mit einem Auslass für das Ablassen des Inhalts des Beutels versehen ist.

## Revendications

1. Poche de stomie comprenant
une première paroi de poche proximale (1) et une seconde paroi de poche distale (2) reliées ensemble le long de leur périphérie externe pour former une cavité destinée à loger des déchets ;
une admission (14) agencée dans la première paroi de poche (1) destinée à recevoir des déchets dans la poche ;
une couche de confort (3) agencée au moins sur la paroi de poche distale (2) ;
la couche de confort (3) comprenant deux panneaux protecteurs (3a, 3b) couvrant chaque côté de la poche et scellés le long de la périphérie, dans laquelle lesdits deux panneaux protecteurs (3a, 3b) butent l'un contre l'autre ou se chevauchent l'un l'autre sans être fixés de façon permanente l'un à l'autre ;
**caractérisée en ce que**
lesdits deux panneaux protecteurs (3a, 3b) butent l'un contre l'autre ou se chevauchent l'un l'autre sans être fixés de façon permanente l'un à l'autre le long d'une ligne orientée longitudinalement (8) sur la poche.

2. Poche selon la revendication 1, dans laquelle la paroi de poche distale (2) est transparente ou translucide.

3. Poche selon la revendication 1 ou 2, dans laquelle les deux panneaux protecteurs (3a, 3b) sont reliés à la paroi de poche distale (2) le long de la périphérie de la poche.

4. Poche selon l'une quelconque des revendications précédentes, dans laquelle les panneaux protecteurs (3a, 3b) de la couche de confort comprennent un film non tissé.

5. Poche selon la revendication 4, dans laquelle les panneaux protecteurs (3a, 3b) comprennent en outre un film opaque comme film interne et le film non tissé comme film externe.

6. Poche selon l'une quelconque des revendications précédentes, dans laquelle la paroi latérale proximale (1) est constituée d'un film opaque, et couverte de préférence d'une couche de confort d'un panneau non tissé.

7. Poche selon l'une quelconque des revendications précédentes, dans laquelle la poche comprend une plaquette adhésive (15) sur la paroi latérale proximale (1) destinée à fixer de façon adhésive la poche à la peau de l'utilisateur.

8. Poche selon la revendication 7, dans laquelle ladite plaquette (15) comprend un trou de départ (17) entouré par une pluralité de lignes guides de coupe (16) destinés à permettre à la personne de couper le trou en une taille d'ouverture souhaitée, où les lignes guides de coupe (16) convergent sensiblement avec le trou de départ (17) au niveau d'une région supérieure du trou (17), c'est-à-dire au niveau d'une radiale correspondant à la ligne centrale longitudinale de la poche.

9. Poche selon l'une quelconque des revendications précédentes, dans laquelle la poche est dotée d'une portion de refoulement (4) avec un refoulement pour vider le contenu de la poche.
